# EUROPÄISCHE PATENTSCHRIFT

(11) **EP 0 751 762 B1**
(45) Veröffentlichungstag und Bekanntmachung des Hinweises auf die Patenterteilung: **06.12.2000**
(21) Anmeldenummer: 95915156.4
(22) Anmeldetag: 24.03.1995
(51) Int. Cl.: A61K 7/48

(54) **HAUTPFLEGEMITTEL**
SKIN CARE PRODUCT
PRODUIT POUR LES SOINS DE LA PEAU

(30) Priorität: 25.03.1994 DE 4410238
(43) Veröffentlichungstag der Anmeldung: 08.01.1997
(73) Patentinhaber: Beiersdorf Aktiengesellschaft, 20245 Hamburg (DE)
(72) Erfinder: HOPPE, Udo, 22397 Hamburg (DE); SAUERMANN, Gerhard, 24649 Wiemersdorf (DE); SCHREINER, Volker, 20259 Hamburg (DE); STEIGER, Klaus-Michael, 8700 Küsnacht (ZH) (CH)
(86) Internationale Anmeldenummer: EP9501116
(87) Internationale Veröffentlichungsnummer: WO9526180

(56) Entgegenhaltungen:
- EP-A- 0 586 106
- WO-A-88/03015
- US-A- 5 378 461

## Beschreibung

Trotz phänomenologischer Verschiedenheit von lichtbedingter Hautalterung und Akne gibt es pathogenetische Gemeinsamkeiten, die in der gestörten Regulation von Zellteilung und -reifung liegen.

Die Haut altert bedingt durch endogene, genetisch determinierte Einflüsse. Exogene Faktoren, wie UV-Licht und chemische Noxen, können kumulativ wirksam sein und beschleunigen die natürlichen Alterungsprozesse. Es kommt zu zahlreichen degenerativen Prozessen, die je nach Größe der Einflußfaktoren u.a. zu folgenden strukturellen Veränderungen und Schäden in Dermis und Epidermis führen (Dermatoheliosis):
a) Rückbildung des mikrovasculären Systems.
b) Schlaffheit und Ausbildung von Falten teilweise aufgrund einer Abnahme und Quervernetzung des Kollagens, Akkumulation der Glucosaminoglycane (Grundsubstanz).
c) Abflachung der Retezapfen. Damit verbunden ist die Verringerung der Fläche zwischen Dermis und Epidermis, über die Stoffe zur Ernährung und Entschlackung der Epidermis ausgetauscht werden.
d) Eingeschränkter regenerativer Turnover in der Epidermis, verbunden mit fehlerhafter Ausbildung der Hornschicht (Verhornungsstörungen), die zur Hautaustrocknung führt.
e) Fehlerhafte Regulation von Zellteilung (Profileration) und Zellreifung (Differenzierung) in der Epidermis, woraus zelluläre Atypen und der Verlust der Polarität resultieren,
f) Lokale Hyper-, Hypo- und Fehlpigmentierungen (Altersflecken).

Bei der Pathogenese von Akne, die besonders im jugendlichen Alter, aber auch in späteren Jahren auftritt, spielen sowohl die Überproduktion von Talgdrüsenlipiden durch überentwickelte Talgdrüsen (Talgdrüsenhyperplasie) als auch Verhornungsstörungen der Talgdrüsenausganges eine wesentliche Rolle. In beiden Fällen kommt es zu einer Anreicherung von Sebumlipiden, die den Nährboden für akneauslösende Bakterien bieten.

Die vorliegende Erfindung betrifft Produkte zur Pflege und Prophylaxe lichtgealterter bzw. zur Ausbildung von Akne neigender Haut sowie zur Behandlung der Folgeschäden der Lichtalterung, insbesondere der unter a) bis f) aufgeführten Phänomene.

Produkte zur Pflege, Prophylaxe und Behandlung lichtgealteter Haut sowie zur prophylaktischen Behandlung von Akne sind an sich bekannt Sie enthalten Retinoide (Vitamin A-Säure und/oder deren Derivate) bzw. Vitamin A und/oder dessen Derivate. Ihre Wirkung auf die Strukturschäden bei Lichtalterung bzw. zur Minderung des Risikos der Akneentstehung ist allerdings umfangmäßig begrenzt. Die Verwendung Vitamin A-Säure-haltiger Produkte bedingt darüber hinaus oft starke erythematöse Hautreizungen. Retinoide sind daher nur in geringen Konzentrationen einsetzbar.

Weiterhin sind kosmetische Zubereitungen mit Coenzym Q-10 aus der DE-A-33 09 850 bekannt, die zur Behandlung von Hautkrankheiten, zur Prophylaxe von dystrophischen und dysmetabolischen Zuständen der Haut und zur Anwendung bei chemischen und physikalischen Respirationsschäden oder bei verzögerter Respiration verbunden mit Alter und Abnutzung geeignet sind.

In der japanischen Offenlegungsschrift 58,180,410 ist die Eignung von Coenzym Q-10 für Kosmetika beschrieben. Es soll den Hautzellmetabolismus aktivieren und die Oxidation unterdrücken. Coenzym Q-10 hat im Resultat eine wichtige Funktion bei der Prävention von Hautschäden durch UV-Strahlen und der Prävention von Hautalterung. Bei 20 bis 40-jährigen wird die Hautrauhigkeit gebessert, indem der Haut Feuchtigkeit gegeben wird.

Ziel der vorliegenden Erfindung war es somit, Wege zu finden, die Nachteile des Standes der Technik zu vermeiden. Insbesondere sollte die prophylaktische Wirkung bei Akne und die restrukturierende Wirkung bei Lichtalterung dauerhaft, nachhaltig und ohne das Risiko von Nebenwirkungen sein.

Erfindungsgemäß werden diese Ziele von Hautpflegepräparaten erreicht, in denen Retinole, Retinale und/oder deren gemeinsame biologische Vorstufe β-Carotin in Kombination mit Ubichinonen und/oder Plastochinonen enthalten sind.

Gegenstand der Erfindung sind topische Zubereitungen mit einem Gehalt an einer Verbindung oder mehreren Verbindungen, ausgewählt aus der Gruppe (A), bestehend aus Retinolen, Retinalen und β-Carotin in Kombination mit einem Gehalt an einer Verbindung oder mehreren Verbindungen, ausgewählt aus der Gruppe (B), bestehend aus Ubichinonen und deren Derivaten und Plastochinonen und deren Derivaten.

Bevorzugt werden Zubereitungen mit einem Gehalt an einer Verbindung oder zwei Verbindungen oder drei Verbindungen, ausgewählt aus der Gruppe (A), kombiniert mit einer Verbindung oder zwei Verbindungen oder drei Verbindungen aus der Gruppe (B).

Die erfindungsgemäßen topischen Zubereitungen können kosmetische oder dermatologische Zubereitungen sein. Sie dienen, wie auch die Wirkstoffe, zur Prophylaxe bei Akne und zur Pflege und Prophylaxe bei Lichtalterung und zur Behandlung lichtgealteter Haut.

Gegenstand der Erfindung ist auch die Verwendung topischer Zubereitungen mit einem Gehalt an einer Verbindung oder mehreren Verbindungen, ausgewählt aus der Gruppe (A), bestehend aus Retinolen, Retinalen und β-Carotin in Kombination mit einem Gehalt an einer Verbindung oder mehreren Verbindungen, ausgewählt aus der Gruppe (B), bestehend aus Ubichinonen und deren Derivaten und Plastochinonen und deren Derivaten zur Pflege und prophylaktischen Behandlung bei Akne und zur Pflege und Prophylaxe bei Lichtalterung und zur Behandlung lichtgealteter Haut.

Gegenstand der Erfindung ist also auch die Verwendung der vorstehenden Zubereitungen und Wirkstoffe für die beschriebenen Zwecke, vorzugsweise aber die Verwendung zur Prophylaxe und Behandlung der folgenden Erscheinungen a) bis f), insbesondere der Dermatoheliosis:
a) Rückbildung des mikrovasculären Systems.
b) Schlaffheit und Ausbildung von Falten teilweise aufgrund einer Abnahme und Quervernetzung des Kollagens, Akkumulation der Glucosaminoglycane (Grundsubstanz).
c) Abflachung der Retezapfen. Damit verbunden ist die Verringerung der Fläche zwischen Dermis und Epidermis, über die Stoffe zur Ernährung und Entschlackung der Epidermis ausgetauscht werden.
d) Eingeschränkter regenerativer Turnover in der Epidermis, verbunden mit fehlerhafter Ausbildung der Hornschicht (Verhornungsstörungen), die zur Hautaustrocknung führt.
e) Fehlerhafte Regulation von Zellteilung (Profileration) und Zellreifung (Differenzierung) in der Epidermis, woraus zelluläre Atypen und der Verlust der Polarität resultieren,
f) Lokale Hyper-, Hypo- und Fehlpigmentierungen (Altersflecken).

Besonders bevorzugt werden die Kombinationen von all-trans Retinol mit Ubichinonen, insbesondere Coenzym Q-9 oder Q-10 und Zubereitungen damit und die Verwendung für die vorstehenden Zwecke.

Die Bezeichnung Retinole im Sinne der Erfindung umfaßt all-trans Retinol, dessen Isomere und deren Derivate. Unter Derivaten des all-trans Retinols oder dessen Isomeren im Sinne der Patentanmeldung sind Verbindungen zu verstehen, in denen z.B. Carbonsäuren, vorzugsweise Fettsäuren, z.B. mit 10 bis 22 Kohlenstoffatomen, insbesondere aber Palmitinsäure, mit dem all-trans Retinol oder dessen Isomeren verestert sind. Unter Isomeren des all-trans Retinols sind Verbindungen zu verstehen, in denen eine oder mehrere Doppelbindungen statt in trans-Konfiguration in cis-Konfiguration vorliegen, insbesondere aber die 11-cis-Konfiguration vorliegt.

Die Bezeichnung Retinale im Sinne der Erfindung umfaßt all-trans Retinal und dessen Isomere. Unter Isomeren des all-trans Retinals sind Verbindungen zu verstehen, in denen eine oder mehrere Doppelbindungen statt in trans-Konfiguration in cis-Konfiguration vorliegen, insbesondere gemeint ist aber das 11-cis-Isomer.

Ubichinone (auch Coenzyme Qₙ) sind eine Gruppe von Substanzen, die an ihrem Chinonring n Isopren-Einheiten kettenförmig gebunden haben (Q₀ - Q₁₀). Ubichinone fungieren bei der biologischen, mitochondralen Oxidation als Elektronenüberträger und spielen so eine bedeutende Rolle beim Energiestoffwechsel der Zellen. Plastochinone sind analoge Verbindungen aus dem Pflanzenbereich, die bei der Photosynthese eine Rolle spielen. In kosmetischen Zubereitungen finden Ubichinone seit langem Anwendung als Antioxidantien zum Schutz oxidationsempfindlicher Substanzen vor Sauerstoff-Radikal-induziertem Zerfall.

Mit 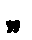 Ubichinone" und 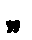 Plastochinone" sind hier auch 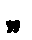 Ubichinone und deren Derivate" und 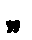 Plastochinone und deren Derivate" gemeint.

Die Ubichinone sind aus der Literatur bekannt (z.B. 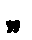 Römpp Chemie Lexikon, Georg Thieme Verlag Stuttgart, New York, 9. Auflage, S. 4784-4785, oder 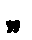 The Merck Index", 11th Edition, Merck & Co., Inc. Rahway, N.Y., USA, Abstr. 9751 (1989)). Sie werden auch als Mitochinone oder Coenzyme Q bezeichnet. Die Anzahl der Isopren-Einheiten in der Seitenkette wird mit n in der Bezeichnung Coenzyme Q-n angegeben, worin n eine ganze Zahl bedeutet Bevorzugt werden Ubichinone oder Coenzyme Q-n mit n=0-12, besonders bevorzugt n=1-12 und insbesondere n=6 bis 10. Gegenstand der Erfindung ist also auch der Chinongrundkörper des Ubichinons ohne Isoprensubstituenten.

Erfindungsgemäße Ubichinone oder deren Derivate sind z.B. auch Alkyl-Ubichinone, insbesondere 6-Alkyl-Ubichinone mit vorzugsweise C₁-C₁₂-Alk yl-Resten. Bevorzugt wird Decyl-Ubichinon, insbesondere 6-Decyl-Ubichinon oder 2,3-Dimethoxy-5-methyl-6-decyl-1,4-benzochinon.

Die Plastochinone sind ebenfalls aus der Literatur bekannt (z.B. 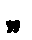 Römpp Chemie Lexikon", Georg Thieme Verlag, Stuttgart, New York, 9. Auflage, S. 3477). Sie sind in der Struktur eng mit den Ubichinonen verwandt und zählen auch zu den Isoprenoid-Chinonen, da sie am Chinonring eine Seitenkette aus Isopren-Einheiten tragen. Bevorzugt werden Plastochinone mit 0 - 12, besonders bevorzugt 1 - 10 und insbesondere 6 bis 10 Isopren-Einheiten in der Seitenkette. Gegenstand der Erfindung ist also auch der Chinongrundkörper des Plastochinons ohne Isoprensubstituenten. Erfindungsgemäße Plastochinone oder deren Derivate sind z.B. auch Alkyl-Plastochinone mit vorzugsweise C₁-C₁₂-Alkyl-Resten. Bevorzugt werden Decyl-Plastochinone, insbesondere 5- oder 6-Decyl-Plastochinon oder 2,3-Dimethyl-5-decyl-1,4-benzochinon.

Ubichinone fungieren bei der biologischen, mitochondralen Oxidation als Elektronenüberträger und spielen so eine bedeutende Rolle beim Energiestoffwechsel der tierischen Zellen. In kosmetischen Zubereitungen finden Ubichinone seit langem Anwendung als Antioxidantien zum Schutz oxidationempfindlicher Substanzen.

Plastochinone sind analoge Verbindungen aus dem Pflanzenbereich, die bei der Photosynthese in den Chloroplasten der pflanzlichen Zellen eine Rolle spielen. Sie unterscheiden sich von den Ubichinonen in drei Substituenten am Chinonring, wobei die zwei Methoxy-Gruppen in den Ubichinonen durch Methylgruppen und eine Methylgruppe durch ein Wasserstoffatom ersetzt sind. Strukturgleich sind jedoch die kettenförmig gebundenen Isopren-Einheiten (vergl. z.B. Pfister und Arntzen, Z. für Naturforschung C34; 996ff, 1979).

Besonders bevorzugt werden die folgenden erfindungsgemäßen Wirkstoffe und Kombinationen damit:
Coenzym Q - 10, Coenzym Q - 9, Coenzym Q - 8, Coenzym Q - 7, Coenzym Q - 6,

Plastochinon mit 10 Isopreneinheiten (auch PQ-10 genannt entsprechend der IUB-Abkürzung PQ für Plastochinone, in der Formel PQ-n soll n die Anzahl der Isopren-Einheiten (0 bis 12) angeben), PQ-9, PQ-8, PQ-7, PQ-6, all-trans Retinol, 11-cis-Retinol.

Es hat sich überraschenderweise gezeigt, daß Retinole und/oder Retinale und/oder β-Carotin in Kombination mit Ubichinonen und/oder Plastochinonen bei der Akne-Prophylaxe bzw. beim Schutz vor Lichtalterung bzw. bei der Reparatur von lichtbedingten Strukturschäden der Haut in synergistischer Weise zusammenwirken, was in signifikanter Weise dem Nachteil des Standes der Technik abhilft.

Die Konzentrationen von Retinolen, Retinalen und/oder β-Carotin in topischen Zubereitungen liegen bevorzugt zwischen 0.01 und 99 Gew.-%.

Die Konzentrationen von Ubichinonen und/oder Plastochinonen in topischen Zubereitungen liegen bevorzugt zwischen 0.001 und 99 Gew.-%.

Vorteilhaft enthalten erfindungsgemäße Hautpflegeprodukte oder Dermatika Kombinationen in folgender Zusammenstellung:
0,01 - 10 Gew.-% Retinole, Retinale und/oder β-Carotin und
0,001 - 10 Gew.-% Ubichinon und/oder Plastochinon

Bevorzugt enthalten Hautpflegepräparate oder Dermatika
0,1 - 1 Gew.-% all-trans Retinol
0,1 -1 Gew.-% Coenzym Q₁₀

Ganz besonders bevorzugt enthalten die Hautpflegeprodukte oder Dermatika
0,3 Gew.-% all-trans Retinol
0,3 Gew.-% Coenzym Q₁₀

Im Rahmen der Anmeldung sind stets Gewichtsprozente bezogen auf 100 Gew.-% Gesamtzusammensetzung des jeweiligen erfindungsgemäßen Hautpflegepräparates oder Dermatikums gemeint.

Die erfindungsgemäßen Wirkstoffkombinationen oder Wirkstoffe können in den topischen Zubereitungen in Mengen von 0,001 bis 99 Gew.-%, z.B. auch in Mengen von 0,001 bis 50 Gew.-% vorliegen, jeweils bezogen auf das Gesamtgewicht der Zubereitungen.

Vorzugsweise können die erfindungsgemäßen Wirkstoffkombinationen oder Wirkstoffe in den topischen Zubereitungen in Mengen von 0,01 bis 10 Gew.-%, insbesondere in Mengen von 0,1 bis 1 Gew.-% vorliegen, jeweils bezogen auf das Gesamtgewicht der Zubereitungen.

In den Kombinationen können die Gewichtsverhältnisse der beiden Komponenten in großen Bereichen schwanken, z.B. im Verhältnis 1 : 100 bis 100 : 1, vorzugsweise im Verhältnis 1 : 10 bis 10 : 1. Sie können z.B. auch im Gewichtsverhältnis 1 : 2 bis
2 : 1 oder 1 : 1 vorliegen.

Im Rahmen der Anmeldung sind stets Gewichtsprozente bezogen auf 100% Gesamtzusammensetzung des jeweiligen erfindungsgemäßen Hautpflegepräparates oder Dermatikums gemeint.

Erfindungsgemäße topische Zubereitungen oder Zusammensetzungen mit den erfindungsgemäßen Kombinationen und Wirkstoffen sind alle gängigen Anwendungsformen, z.B. Cremes (W/O, O/W, W/O/W), Gele, Lotionen, Milchen.

Die erfindungsgemäßen topischen Zubereitungen können als flüssige, pastöse oder feste Zubereitungen formuliert werden, beispielsweise als wäßrige oder alkoholische Lösungen, wäßrige Suspensionen, Emulsionen, Salben, Cremes, Öle, Pulver oder Stifte. In Abhängigkeit von der gewünschten Formulierung können die Wirkstoffe in pharmazeutische und kosmetische Grundlagen für topische Applikationen eingearbeitet werden, die als weitere Komponenten beispielsweise Ölkomponenten, Fett und Wachse, Emulgatoren, anionische, kationische, ampholyitsche, zwitterionische und/oder nichtionogene Tenside, niedere ein- und mehrwertige Alkohole, Wasser, Konservierungsmittel, Puffersubstanzen, Verdickungsmittel, Duftstoffe, Farbstoffe und Trübungsmittel enthalten. Vorteilhaft können die erfindungsgemäßen Wirkstoffe auch in transdermalen therapeutischen Systemen, insbesondere kubischen Systemen verwendet werden.

Es ist ferner von Vorteil, den Zubereitungen Antioxidantien (z.B. alpha-Tocopherol, Vitamin E und C, Imidazole, alpha-Hydroxycarbonsäuren (z.B. Äpfelsäure, Glycolsäure, Gluconsäure, Salicylsäure sowie deren Derivate) und/oder Eisenkomplexbildner (z.B. EDTA, alpha-Hydroxyfettsäuren) und/oder bekannte UV-Lichtschutzfilter in Mengen von z.B. 0,1 bis 10 Gewichtsprozenten zuzusetzen, um die Stabilität der oxidationsempfindlichen Wirkstoffe zu gewährleisten.

Auch ist es vorteilhaft, den Zubereitungen insbesondere 0,01 - 10 Gewichtsprozente an Stoffen bzw. Stoffkombinationen des aeroben zellulären Energiestoffwechsels (z.B. zelluläre Energieüberträger (wie Kreatin, Guanin. Guanosin, Adenin, Adenosin, Nicotin, Nicotinamid, Riboflavin), Coenzyme (z.B. Pantothensäure, Panthenol, Liponsäure, Niacin), Hilfsfaktoren (z.B. L-Carnitin, Uridin), Substrate (z.B. Hexosen, Pentosen, Fettsäuren) und intermediäre Stoffwechselprodukte (z.B. Zitronensäure, Pyruvat) und/oder Glutathion zuzusetzen.

Vorteilhaft können erfindungsgemäße Zubereitungen außerdem Substanzen enthalten, die UV-Strahlung im UVA- und/oder im UVB-Bereich absorbieren, wobei die Gesamtmenge der Filtersubstanzen z.B. 0,1 Gew.-% bis 30 Gew.-%, vorugsweise 0,5 bis 10 Gew.-%, insbesondere 1,0 bis 6,0 Gew.-% beträgt, bezogen auf das Gesamtgewicht der Zubereitungen, um kosmetische Zubereitungen zur Verfügung zu stellen, die die Haut vor dem gesamten Bereich der ultravioletten Strahlung schützen. Sie können auch als Sonnenschutzmittel für die Haut dienen. In den Zubereitungen wirken die UV-Absorber gegenüber den Wirkstoffen als Antioxidantien.

Enthalten die erfindunsgemäßen Emulsionen UVB-Filtersubstanzen, können diese öllöslich oder wasserlöslich sein. Erfindungsgemäß vorteilhafte öllösliche UVB-Filter sind z.B.:
3-Benzylidencampher-Derivate, vorzugsweise 3-(4-Methylbenzyliden)campher, 3-Benzylidencampher.

Vorteilhaft wasserlösliche UVB-Filter sind z.B.:
Salze der 2-Phenylbenzimidazol-5-sulfonsäure wie ihr Natrium-, Kalium- oder ihr Triethanolammonium-Salz, sowie die Sulfonsäure selbst.

Es kann auch von Vorteil sein, erfindungsgemäße Wirkstoffkombinationen mit UVA-Filtern zu kombinieren, die bisher üblicherweise in kosmetischen Zubereitungen enthalten sind. Bei diesen Substanzen handelt es sich vorzugsweise um Derivate des Dibenzoylmethans, insbesondere um 1-(4'-tert.Butylphenyl)-3-(4'-methoxyphenyl)propan-1,3-dion und um 1-Phenyl-3-(4'isopropylphenyl)propan-1,3dion. Auch diese Kombinationen bzw. Zubereitungen, die diese Kombinationen enthalten, sind Gegenstand der Erfindung. Es können die für die UVB-Kombination verwendeten Mengen eingesetzt werden.

Gegenstand der Erfindung sind also auch die Kombinationen der erfindungsgemäßen Wirkstoffe, insbesondere in den topischen Zubereitungen, mit Antioxydantien, Stoffen des aeroben zellulären Energiestoffwechsels und/oder UV-Absorbern, durch die sich z.B. die Stabilität und die Wirkung der Zubereitung verbessern läßt.

Die vorstehend aufgeführten Beispiele für kombinierbare Wirkstoffe aus den angegebenen Wirkstoffgruppen dienen dazu, die Erfindung zu beschreiben, ohne daß beabsichtigt ist, die Erfindung auf diese Beispiele zu beschränken.

Darüber hinaus können schützende Formulierungsformen angewendet werden, wobei die erfindungsgemäßen Stoffe z.B. in Liposomen, Micellen, Nanosphären usw. aus z.B. hydrierten Amphiphilen, wie z.B. Ceramiden, Fettsäuren, Sphingomyelin und Phosphoglyceriden bzw. in Zyklodextrane eingeschossen (verkapselt) werden. Weiterer Schutz kann durch die Verwendung von Schutzgas (z.B. N₂, CO₂) bei der Formulierung und die Verwendung gasdichter Verpackungsformen erreicht werden.

Weitere Hilfs- und Zusatzstoffe können wasserbindende Stoffe, Verdicker, Füllstoffe, Parfüm, Farbstoffe, Emulgatoren, Wirkstoffe wie Vitamine, Konservierungsmittel, Wasser und/oder Salze sein.

Bei der Verarbeitung der Wirkstoffe und anderer oxidationsempfindlicher Stoffe sollte die Temperatur nicht über 40°C liegen. Ansonsten sind die üblichen Maßregeln zu beachten, die dem Fachmann bekannt sind.

Die erfindungsgemäßen Stoffgruppen lassen sich so in alle kosmetischen Grundlagen einarbeiten. Grundsätzlich sind allerdings W/O- und O/W-und W/O/W-Emulsionen bevorzugt. Besonders vorteilhaft können erfindungsgemäße Kombinationen in Pflegeprodukte wie beispielsweise O/W-Cremes, W/O-Cremes, O/W-Lotionen, W/O-Lotionen usw. eingesetzt werden.

Alle Mengenangaben, Prozentangaben oder Teile beziehen sich, soweit nicht anders angegeben, auf das Gewicht, insbesondere auf das Gesamtgewicht der Zubereitungen oder der jeweiligen Mischung.

Die folgenden Beispiele dienen dazu, die Erfindung zu beschreiben, ohne daß beabsichtigt ist, die Erfindung auf diese Beispiele zu beschränken.

| Kombination | A | B | C | D | E | F |
|---|---|---|---|---|---|---|
| all-trans Retinol | 50 | 25 | 10 | 90 | 40 | - |
| all-trans Retinal | - | 25 | - | - | - | 50 |
| all-trans Retinyl-palmitat | - | - | - | - | 20 | - |
| Coenzym Q₆ | - | 25 | - | - | - | 40 |
| Coenzym Q₁₀ | 50 | 25 | 90 | 10 | 40 | 10 |
| | 100 | 100 | 100 | 100 | 100 | 100 |

Die Teile und Zahlenangaben beziehen sich auf Gewichtsteile.

### Beispiel I

### (mit Kombination A)

### Hautcreme vom W/O-Typ

| | Gew.-Teile |
|---|---|
| Vaseline DAB 9 | 13 |
| Glycerin DAB 9 | 6,3 |
| Wasser VES | 34,4 |
| Paraffinöl (Mineralöl 5E,Shell) | 31 |
| Cetearylalkohol/PEG-40-Castor Oil/Natriumcetearyl-sulfat (Emulgade F, Henkel KGaA) | 2,5 |

In die 75°C warme Fettphase werden 0,3 Teile Coenzym Q₁₀ in 3 Teilen Paraffinöl gelöst eingearbeitet. Die Fettphase wir sodann zu der 75°C warmen Wasserphase gegeben, verrührt und homogenisiert, bis eine gleichmäßige hellgelbe Creme entstanden ist. 0,3 Teile all-trans Retinol werden bei Raumtemperatur in weiteren 3,2 Teilen Paraffinöl gelöst und in die abgekühlte Creme eingerührt.

Beispiel I hat folgende endgültige Zusammensetzung:

| | Gew.-Teile |
|---|---|
| Vaseline DAB 9 | 13 |
| Glycerin DAB 9 | 6,3 |
| Wasser VES | 34,4 |
| Paraffinöl (Mineralöl 5E,Shell) | 43,2 |
| Cetearylalkohol/PEG-40-Castor Oil/Natriumcetearyl-sulfat (Emulgade F, Henkel KGaA) | 2,5 |
| all-trans Retinol | 0,3 |
| Coenzym Q₁₀ | 0,3 |
| | 100 |

### Beispiel II

### (mit Kombination B)

### Hautcreme vom W/O-Typ

| | Gew.-Teile |
|---|---|
| PEG-1 Glyceryl-Oleostearat + Paraffinwachs | 8 |
| Vaseline DAB | 2,8 |
| Paraffinwachs/Paraffin | 1,8 |
| Ceresin | 2,2 |
| Octyldodecanol | 10 |
| (Eutanol G, Henkel KGaA) Propylenglycol | 1 |
| Glycerin | 1 |
| Magnesiumsulfat | 0,7 |
| Wasser VES (vollentsaltzt) | 59,7 |
| Summe Additive (Parfüm, Konservierung, Stabilisation) | 0,8 |

In die 75°C warme Fettphase werden 0,2 Teile Coenzym Q₁₀ und 0,2 Teile Coenzym Q₆ in 6 Teilen Paraffinöl gelöst eingearbeitet. Die Fettphase wir sodann zu der 75°C warmen Wasserphase gegeben, verrührt und homogenisiert, bis eine gleichmäßige hellgelbe Creme entstanden ist. 0,2 Teile all-trans Retinol und 0,2 Teile all-trans Retinal werden bei Raumtemperatur in weiteren 5,5 Teilen Paraffinöl gelöst und in die abgekühlte Creme eingerührt.

Beispiel II hat folgende endgültige Zusammensetzung:

| | Gew.-Teile |
|---|---|
| PEG-1 Glyceryl-Oleostearat + Paraffinwachs | 8 |
| Vaseline DAB | 2,8 |
| Paraffinwachs/Paraffin | 1,8 |
| Paraffinöl (Mineralöl 5E,Shell) | 11,5 |
| Ceresin | 2,2 |
| Octyldodecanol | 10 |
| all-trans Retinol | 0,2 |
| all-trans Retinal | 0,2 |
| Coenzym Q₆ | 0,2 |
| Coenzym Q₁₀ | 0,2 |
| Propylenglycol | 1 |
| Glycerin | 1 |
| Magnesiumsulfat | 0,7 |
| Wasser VES | 59,4 |
| Summe Additive (Parfüm, Konservierung, Stabilisation) | 0,8 |
| | 100 |

### Beispiel III

### (mit Kombination C)

### Hautcreme vom O/W-Typ

| | Gew.-Teile |
|---|---|
| Octyldodecanol (Eutanol G, Henkel KGaA) | 9,3 |
| Cetearylalkohol/PEG-40-Castor Oil/Natriumcetearylsulfat (Emulgade F, Henkel KGaA) | 3,7 |
| Wasser VES | 73,7 |
| Glycerin DAB 9 | 4,6 |

In die 75°C warme Fettphase werden 0,9 Teile Coenzym Q₁₀ in 4 Teilen Paraffinöl gelöst eingearbeitet Die Fettphase wir sodann zu der 75°C warmen Wasserphase gegeben, verrührt und homogenisiert, bis eine gleichmäßige hellgelbe Creme entstanden ist 0,1 Teile all-trans Retinol werden bei Raumtemperatur in weiteren 3,7 Teilen Paraffinöl gelöst und in die abgekühlte Creme eingerührt.

Beispiel III hat folgende entgültige Zusammensetzung:

| | |
|---|---|
| Octyldodecanol (Eutanol G, Henkel KGaA) | 9,3 |
| Cetearylalkohol/PEG-40-Castor Oil/Natriumcetearylsulfat (Emulgade F, Henkel KGaA) | 3,7 |
| Wasser VES | 73,7 |
| Glycerin DAB 9 | 4,6 |
| Paraffinöl (Mineralöl 5E, Shell) | 7,7 |
| Coenzym Q₁₀ | 0,9 |
| all-trans Retinol | 0,1 |
| | 100 |

### Beispiel IV

### (mit Kombination D)

### O/W-Lotion

| | Gew.-Teile |
|---|---|
| Steareth-2 | 3 |
| Steareth-21 | 2 |
| Cetearylalkohol/PEG-40-Castor Oil/Natriumcetearylsulfat (Emulgade F, Henkel KGaA) | 2,5 |
| Propylenglycol | 1 |
| Glycerin | 1 |
| Wasser VES | 74,3 |
| Summe Additive (Parfüm, Konservierung, Stabilisation) | 0,8 |

In die 75°C warme Fettphase werden 0,1 Teile Coenzym Q₁₀ in 5,2 Teile Paraffinöl gelöst eingearbeitet. Die Fettphase wir sodann zu der 75°C warmen Wasserphase gegeben, verrührt und homogenisiert, bis eine gleichmäßige hellgelbe Lotion entstanden ist. 0,9 Teile alt-trans Retinol werden bei Raumtemperatur in weiteren 9 Teilen Paraffinöl gelöst und in die abgekühlte Lotion eingerührt.

Beispiel IV hat folgende endgültige Zusammensetzung:

| | Gew.-Teile |
|---|---|
| Steareth-2 | 3 |
| Steareth-21 | 2 |
| Cetearylalkohol/PEG-40-Castor Oil/Natriumcetearylsulfat (Emulgade F, Henkel KGaA) | 2,5 |
| Paraffinöl (Mineralöl 5E, Shell) | 14,4 |
| Propylenglycol | 1 |
| Coenzym Q₁₀ | 0,1 |
| all-trans Retinol | 0,9 |
| Glycerin | 1 |
| Wasser VES | 74,3 |
| Summe Additive (Parfüm, Konservierung, Stabilisation) | 0,8 |
| | 100 |

### Beispiel V

### (mit Kombination E)

### O/W-Lotion

| | Gew.-Teile |
|---|---|
| Octyldodecanol (Eutanol G, Henkel KGaA) | 5,6 |
| Cetearylalkohol/PEG-40-Castor Oil/Natriumcetearylsulfat (Emulgade F, Henkel KGaA) | 8,9 |
| Cetearylisononanoat (Cetiol 5N, Henkel KGaA) | 7,5 |
| Wasser VES | 62,3 |
| Glycerin DAB 9 | 4,7 |

In die 75°C warme Fettphase werden 0,4 Teile Coenzym Q₁₀ in 6 Teilen Paraffinöl gelöst eingearbeitet. Die Fettphase wir sodann zu der 75°C warmen Wasserphase gegeben, verrührt und homogenisiert, bis eine gleichmäßige hellgelbe Lotion entstanden ist. 0,4 Teile all-trans Retinol und 0,2 Teile Retinylpalmitat werden bei Raumtemperatur in weiteren 4 Teilen Paraffinöl gelöst und in die abgekühlte Lotion eingerührt.

Beispiel V hat folgende endgültige Zusammensetzung:

| | Gew.-Teile |
|---|---|
| Octyldodecanol (Eutanol G, Henkel KGaA) | 5,6 |
| Cetearylalkohol/TEG-40-Castor Oil/Natriumcetearylsulfat (Emulgade F, Henkel KGaA) | 8,9 |
| Cetearylisononanoat (Cetiol 5N, Henkel KGaA) | 7,5 |
| Wasser VES | 62,3 |
| Glycerin DAB 9 | 4,7 |
| Paraffinöl (Mineralöl 5E, Shell) | 10 |
| Coenzym Q₁₀ | 0,4 |
| all-trans Retinol | 0,4 |
| all trans Retinylpalmitat | 0,2 |
| | 100 |

### Beispiel VI

### (mit Kombination F)

### Hautöl

| | Gew.-Teile |
|---|---|
| Glyceryltricaprylat (Miglyol 812, Dynamit Nobel) | 21 |
| Hexyllaurat (Cetiol A, Henkel KGaA) | 20 |
| Octylstearat (Cetiol 886, Henkel KGaA | 20 |
| Paraffinöl (Mineralöl 5E, Shell) | 35 |
| all-trans Retinal | 2 |
| Coenzym Q₆ | 1,6 |
| Coenzym Q₁₀ | 0,4 |
| | 100 |

Die Komponenten werden bei 25°C verrührt, bis eine gleichmäßige, klare Mischung entstanden ist.

## Patentansprüche

1. Topische Zubereitungen mit einem Gehalt an einer Verbindung oder mehreren Verbindungen, ausgewählt aus der Gruppe (A), bestehend aus Retinolen, Retinalen und β-Carotin in Kombination mit einem Gehalt an einer Verbindung oder mehreren Verbindungen, ausgewählt aus der Gruppe (B), bestehend aus Ubichinonen und deren Derivaten und Plastochinonen und deren Derivaten.

2. Zubereitungen gemäß Anspruch 1 mit einem Gehalt an einer Verbindung oder zwei Verbindungen oder drei Verbindungen, ausgewählt aus der Gruppe (A), kombiniert mit einer Verbindung oder zwei Verbindungen oder drei Verbindungen aus der Gruppe (B).

3. Zubereitungen gemäß Anspruch 1, dadurch gekennzeichnet, daß sie Retinole, insbesondere all-trans Retinol enthalten.

4. Zubereitungen gemäß Anspruch 1, dadurch gekennzeichnet, daß sie mindestens ein Ubichinon enthalten.

5. Zubereitungen gemäß Anspruch 1, dadurch gekennzeichnet, daß sie all-trans Retinol und Coenzym Q-10 enthalten.

6. Zubereitungen gemäß Anspruch 1, dadurch gekennzeichnet, daß sie Ubichinone oder Plastochinone mit 0 bis 12 Isopreneinheiten und/oder gegebenenfalls Alkyl-Reste besitzen.

7. Zubereitungen gemäß Anspruch 1, dadurch gekennzeichnet, daß die Ubichinone oder Plastochinone 9 oder 10 Isopreneinheiten besitzen.

8. Kosmetische Verwendung topischer Zubereitungen mit einem Gehalt an einer Verbindung oder mehreren Verbindungen. ausgewählt aus der Gruppe (A), bestehend aus Retinolen, Retinalen und β-Carotin in Kombination mit einem Gehalt an einer Verbindung oder mehreren Verbindungen, ausgewählt aus der Gruppe (B), bestehend aus Ubichinonen und deren Derivaten und Plastochinonen und deren Derivaten zur Pflege und prophylaktischen Behandlung bei Akne und zur Pflege und Prophylaxe bei Lichtalterung und zur Behandlung lichtgealteter Haut.

9. Zubereitungen gemäß Anspruch 1, dadurch gekennzeichnet, daß sie Antioxidantien, Stoffe des aeroben zellulären Energiestoffwechsels und/oder UV-Absorber enthalten.

10. Zubereitungen gemäß Anspruch 1, dadurch gekennzeichnet, daß sie kosmetische oder dermatologische Zubereitungen, insbesondere W/O-, O/W- oder W/O/W-Emulsionen sind.

## Claims

1. Topical formulations containing one or more compounds chosen from the group (A) consisting of retinols, retinals and B-carotene in combination with a content of one or more compounds chosen from the group (B) consisting of ubiquinones and derivatives thereof and plastoquinones and derivatives thereof.

2. Formulations according to Claim 1 containing one, two or three compounds chosen from the group (A) combined with one, two or three compounds from the group (B).

3. Formulations according to Claim 1, characterized in that they comprise retinols, in particular all-trans retinol.

4. Formulations according to Claim 1, characterized in that they comprise at least one ubiquinone.

5. Formulations according to Claim 1, characterized in that they comprise retinol and coenzyme Q-10.

6. Formulations according to Claim 1, characterized in that they have ubiquinones or plastoquinones having from 0 to 12 isoprene units and/or optionally alkyl radicals.

7. Formulations according to Claim 1, characterized in that the ubiquinones or plastoquinones have 9 or 10 isoprene units.

8. Cosmetic use of topical formulations containing one or more compounds chosen from the group (A) consisting of retinols, retinals and B-carotene in combination with a content of one or more compounds chosen from the group (B) consisting of ubiquinones and derivatives thereof and plastoquinones and derivatives thereof for the care and prophylactic treatment in cases of acne and for the care and prophylaxis in cases of light-ageing and for the treatment of light-aged skin.

9. Formulations according to Claim 1, characterized in that they comprise antioxidants, substances of aerobic cellular energy metabolism and/or UV absorbers.

10. Formulations according to Claim 1, characterized in that they are cosmetic or dermatological formulations, in particular W/O, O/W or W/O/W emulsions.

## Revendications

1. Préparations topiques ayant une teneur en un composé ou plusieurs composés choisi(s) dans le groupe (A) constitué par les rétinols, les rétinals et le β-carotène, en association avec une teneur en un composé ou plusieurs composés choisi(s) dans le groupe (B) constitué par les ubiquinones et leurs dérivés et les plastoquinones et leurs dérivés.

2. Préparations selon la revendication 1, ayant une teneur en un composé ou deux composés ou trois composés, choisi(s) dans le groupe (A), en association avec un composé ou deux composés ou trois composés choisi(s) dans le groupe (B).

3. Préparations selon la revendication 1, caractérisées en ce qu'elles contiennent des rétinols, en particulier du rétinol tout-trans.

4. Préparations selon la revendication 1, caractérisées en ce qu'elles contiennent une ubiquinone.

5. Préparations selon la revendication 1, caractérisées en ce qu'elles contiennent du rétinol tout-trans et de la coenzyme Q-10.

6. Préparations selon la revendication 1, caractérisées en ce qu'elles contiennent des ubiquinones ou des plastoquinones comportant de 0 à 12 unités isoprène et/ou éventuellement des radicaux alkyle.

7. Préparations selon la revendication 1, caractérisées en ce que les ubiquinones ou les plastoquinones comportent 9 ou 10 unités isoprène.

8. Utilisation cosmétique de préparations topiques ayant une teneur en un composé ou plusieurs composés choisi(s) dans le groupe (A) constitué par les rétinols, les rétinals et le β-carotène, en association avec une teneur en un composé ou plusieurs composés choisi(s) dans le groupe (B) constitué par les ubiquinones et leurs dérivés et les plastoquinones et leurs dérivés, pour le soin et le traitement prophylactique dans l'acné et pour le soin et la prophylaxie dans le photovieillissement et pour le traitement de la peau présentant un vieillissement photo-induit.

9. Préparations selon la revendication 1, caractérisées en ce qu'elles contiennent des antioxydants, des substances du métabolisme énergétique cellulaire aérobie et/ou des absorbeurs d'UV.

10. Préparations selon la revendication 1, caractérisées en ce qu'elles sont des préparations cosmétiques ou dermatologiques, en particulier des émulsions E/H, H/E ou E/H/E.
